# EUROPÄISCHE PATENTSCHRIFT

(11) **EP 0 962 444 B1**
(45) Veröffentlichungstag und Bekanntmachung des Hinweises auf die Patenterteilung: **02.10.2002**
(21) Anmeldenummer: 99110273.2
(22) Anmeldetag: 27.05.1999
(51) Int. Cl.: C07C 67/54, C07C 69/15

(54) **Verfahren zur Aufarbeitung von rohem, flüssigem Vinylacetat**
Process for treatment of crude liquid vinyl acetate
Procédé de traitement d'acétate de vinyle brut liquide

(30) Priorität: 05.06.1998 DE 19825254
(43) Veröffentlichungstag der Anmeldung: 08.12.1999
(73) Patentinhaber: Celanese Chemicals Europe GmbH, 60439 Frankfurt am Main (DE)
(72) Erfinder: Hess, Stefan Dr., 64521 Gross-Gerau (DE); Mark, Michael Dr., 60529 Frankfurt (DE); Meilchen, Melchior A. Dr., Houston, TX 77062 (US); Stamm, Johann, 65929 Frankfurt (DE); Vernaleken, Thomas, 64331 Weiterstadt (DE); Wagner, Martin, 65929 Frankfurt (DE)

(56) Entgegenhaltungen:
- EP-A- 0 072 484
- EP-A- 0 423 658
- DE-A- 2 132 299

## Beschreibung

Die vorliegende Erfindung betrifft ein Verfahren zur Aufarbeitung von rohem, flüssigem Vinylacetat, das Essigsäure und Wasser, sowie Ethylacetat und gegebenenfalls noch weitere Verunreinigungen enthält.

Bei Verfahren zur Herstellung von Vinylacetat erhält man häufig ein flüssiges, rohes Vinylacetat, das im wesentlichen noch Essigsäure und Wasser sowie Ethylacetat und gegebenenfalls noch weitere Verunreinigungen enthält. Ein derartiges Produkt kann z.B. bei der Herstellung von Vinylacetat durch Umsetzung von Ethylen und Essigsäure und Sauerstoff in der Gasphase bei erhöhter Temperatur und normalem oder erhöhtem Druck in Gegenwart eines Katalysators erhalten werden, indem man die den Reaktor verlassenden Gase abkühlt und/oder mit Essigsäure wäscht. Eine derartige Herstellung von Vinylacetat wird im allgemeinen mit überschüssiger Essigsäure durchgeführt, wobei die nicht umgesetzte Essigsäure im Kreislauf in die Vinylacetatherstellung zurückgeführt wird.

Eine Aufarbeitung von aus solchem Herstellungsverfahren zugänglichem, rohem, flüssigem Vinylacetat, das noch Essigsäure und Wasser, sowie Ethylacetat und gegebenenfalls noch weitere Verunreinigungen enthält, ist in der DE - AS 1 768 412 beschrieben. Bei diesem Verfahren wird das rohe Vinylacetat azeotrop destilliert, durch Rückführung des durch Kondensation und Phasentrennung des Kopfproduktes erhältlichen Vinylacetats die Hauptmenge des Wassers zusammen mit Vinylacetat über Kopf abgezogen und soviel Vinylacetat zurückgeführt, daß als Sumpfprodukt eine Essigsäure erhalten wird, die etwa 0,5 bis 6 Gew.-% Wasser enthält. Gegebenenfalls kann aus dieser Destillation ein flüssiger Seitenstrom entnommen werden, in dem Ethylacetat angereichert ist. Das nach Kondensation und Phasentrennung des gasförmigen Kopfproduktes erhältliche Vinylacetat enthält etwa 100 bis 500 Gew.- ppm Ethylacetat.

Ein weiteres bekanntes Verfahren zur Aufarbeitung von rohem Vinylacetat wird in der DE-PS 1 282 014 und der DE-PS 1 668 063 beschrieben. Hierbei wird in einer ersten Destillationskolonne das Wasser als azeotropes Gemisch und die niedriger als Vinylacetat siedenden Nebenprodukte über Kopf destilliert und der praktisch wasserfreie Sumpf in einer zweiten Destillationskolonne destilliert, wobei man am Kopf der zweiten Destillationskolonne Vinylacetat, aus einer oder mehreren Anreicherungszonen zwischen Kopf und Sumpf mindestens den größten Teil der höher als Vinylacetat siedenden Nebenprodukte und unterhalb der untersten Anreicherungszone oder als Sumpf die Essigsäure und gegebenenfalls den Rest der Nebenprodukte entnimmt. Das in der ersten Kolonne nach Kondensation und Phasentrennung anfallende Vinylacetat wird im allgemeinen vollständig in die erste Kolonne zurückgeführt und Vinylacetat nur vom Kopf der zweiten Kolonne entnommen. Nach der Lehre von DE-PS-1 668 063 kann bei einer Druckerhöhung in der ersten Destillationskolonne von Normaldruck auf 1 atü bei etwa der halben Rücklaufmenge die gleiche Wassermenge am Kopf der zweiten Kolonne ausgetragen werden. Angaben zum Wassergehalt des gasförmigen Kopfprodukts der ersten Kolonne werden nicht gemacht, der Ethylacetatgehalt im abgetrennten Vinylacetat beträgt etwa 1000 ppm.

In der DE-OS 2 943 985 wird ein Verfahren zur Abtrennung von Wasser aus Gemischen mit Vinylacetat und Essigsäure beschrieben, bei dem ein durch Abkühlung des aus der Reaktionszone austretenden Gasgemisches erhaltenes Kondensat, das die Hauptmenge der Essigsäure, des Vinylacetats und des Wassers enthält, und eine durch Adsorption mit Essigsäure erhaltene Lösung, die das nicht kondensierte, restliche Vinylacetat und Wasser enthält, auf verschiedene Böden in eine Destillationskolonne gegeben werden. Das Kopfprodukt kann dann ca. 3 bis 5 Gew.-% Wasser enthalten. Ob Ethylacetat enthaltenes rohes Vinylacetat so aufgearbeitet werden kann und welche Gehalte an Ethylacetat dann im abgetrennten Vinylacetat vorkommen, ist· in dieser DE-OS nicht offenbart.

Schließlich wird in der DE-AS 1 618 240 eine Abtrennung von Ethylacetat aus Vinylacetat beschrieben, bei der eine Extraktivdestillation mit Wassser als Schleppmittel durchgeführt wird. Mit einem Gewichtsverhältnis von Wasser zu organischer Rohbeschickung, bestehend aus 99,8 Gew.-% Vinylacetat und 0,2 Gew.-% Ethylacetat, von 0,19:1 und bei einem Rücklaufverhältnis von ungefähr 3:1 wird ein Überkopfstrom aus gereinigtem Vinylacetat, der weniger als 500 Gew.-ppm Ethylacetat,bezogen auf Vinylacetat, enthält, abgezogen. Das eingesetzte Vinylacetat enthält dabei jedoch keine nennenswerten Mengen Wasser und/oder Essigsäure.

Außerdem ist aus US-A-3,404,177 ein Verfahren zur Abtrennung von Vinylacetat, Wasser, Essigsäure und gegebenenfalls Acetaldehyd aus gasförmigen Reaktionsgemischen bekannt. Solche Reaktionsgemische enthalten auch noch Ethylen, Sauerstoff und CO₂. Ethylacetat und seine Abtrennung wird in dieser Schrift nicht offenbart. Effekte, die mit einer Wassereinspeisung oder Azeotropbildung einhergehen, ebenfalls nicht.

Schließlich offenbart EP-B-0 072 484 ein Verfahren zur Aufarbeitung von rohem, flüssigem Vinylacetat, das Essigsäure und Wasser, sowie Ethylacetat und gegenbenenfalls weitere kleine Mengen an Verunreinigungen enthält, durch Destillation, wobei man als Kopfprodukt ein im wesentlichen Vinylacetat und Wasser enthaltendes Gemisch und als Sumpfprodukt im wesentlichen Essigsäure erhält und einen Seitenstrom entnimmt, in dem Ethylacetat angereichert ist, und wobei man das Kopfprodukt kondensiert und nach Phasentrennung einen Teil der Vinylacetat-Phase als Rücklauf in die Destillation zurückführt, das dadurch gekennzeichnet ist, daß man in die Destillation oberhalb der Zugabestelle des rohen, flüssigen Vinylacetats 0,1 bis 5 Gew.-% Wasser, bezogen auf das eingesetzte rohe, flüssige Vinylacetat, einleitet. Das bekannte Verfahren führt zu einer ausreichenden Entwässerung in der Destillation und zu einer Verringerung des Ethylacetatgehaltes in dem als Kopfprodukt erhaltenen Vinylacetat.

Aus DE-OS-2 132 299 ist bekannt, daß flüssige Vinylacetat und Ethylacetat enthaltende Gemische einer Extraktivdestillation mit Essigsäure als Schleppmittel unterzogen werden können. Das offenbarte Verfahren führt zu einer Verringerung des Ethylacetatgehaltes in dem am Kolonnenkopf abgezogenen Vinylacetat auf ca. 200 Gew.-ppm. Nach der Lehre von DE-OS-2 132 299 müssen jedoch 60 g Essigsäure pro 100 g eingesetztem rohem, flüssigem Vinylacetat zugespeist werden.

Überraschenderweise wurde nun gefunden, daß sich der Ethylacetatgehalt in den die Destillation am Kopf verlassenden Gasen verringern und in dem die Destillation verlassenden Seitenstrom erhöhen läßt und der Vinylacetat-Austrag über diesen Seitenstrom verringert wird, wenn man in die Destillationskolonne oberhalb der Zugabestelle des rohen, flüssigen Vinylacetats 0,1 bis 5 Gew.-% Wasser, bezogen auf das eingesetzte rohe, flüssige Vinylacetat, und 0,1 bis 60 Gew.-% Essigsäure, bezogen auf das eingesetzte rohe, flüssige Vinylacetat, einleitet.

Der Gegenstand der vorliegender Erfindung betrifft demnach ein Verfahren zur Aufarbeitung von rohem, flüssigem Vinylacetat, das Essigsäure und Wasser, sowie Ethylacetat und gegebenenfalls weitere kleinere Mengen anderer Verunreinigungen enthält, durch Destillation, wobei man als Kopfprodukt ein im wesentlichen Vinylacetat und Wasser enthaltendes Gemisch und als Sumpfprodukt im wesentlichen Essigsäure erhält und einen Seitenstrom entnimmt, in dem Ethylacetat angereichert ist, und wobei man das Kopfprodukt kondensiert und nach Phasentrennung einen Teil der Vinylacetat-Phase als Rücklauf in die Destillationskolonne zurückführt, dadurch gekennzeichnet, daß man in die Destillationskolonne oberhalb der Zugabestelle des rohen, flüssigen Vinylacetats 0,1 bis 5 Gew.-% Wasser, bezogen auf das eingesetzte rohe, flüssige Vinylacetat, und 0,1 bis 60 Gew.-% Essigsäure, bezogen auf das eingesetzte rohe, flüssige Vinylacetat, einleitet.

Zum Einsatz in das erfindungsgemäßige Verfahren ist beispielsweise rohes, flüssiges Vinylacetat geeignet, das etwa 12 bis 30 Gew.-% Vinylacetat, etwa 3 bis 10% Gew.-% Wasser, etwa 0,01 bis 0,2 Gew.-% Ethylacetat und ergänzend auf 100 Gew.-% Essigsäure enthält. Weitere Verunreinigungen, beispielsweise Diacetate, Polymere, Acetaldehyd und/oder Methylacetat können ebenfalls in geringen Mengen, beispielsweise jeweils in Mengen von unter 0,5 Gew.-% vorhanden sein. Solche Verunreinigungen können bei der Vinylacetat-Herstellung gebildet oder schon mit den Einsatzprodukten in die Vinylacetat-Herstellung eingebracht worden sein.

Ein für den Einsatz in das erfindungsgemäße Verfahren geeignetes rohes, flüssiges Vinylacetat kann auf verschiedene Weise erhalten werden. Beispielsweise kann man in bekannter Weise Ethylen, Sauerstoff oder Sauerstoff enthaltende Gase und Essigsäure in der Gasphase bei erhöhter Temperatur und normalem oder erhöhtem Druck, an Edelmetallen und/oder deren Verbindungen enthaltenden Katalysatoren umsetzen und die aus dieser Umsetzung kommenden Gase abkühlen und/oder waschen, beispielsweise mit Essigsäure oder Essigsäure enthaltenden Waschflüssigkeiten, und das so erhältliche rohe, flüssige Vinylacetat in das erfindungsgemäße Verfahren einsetzen.

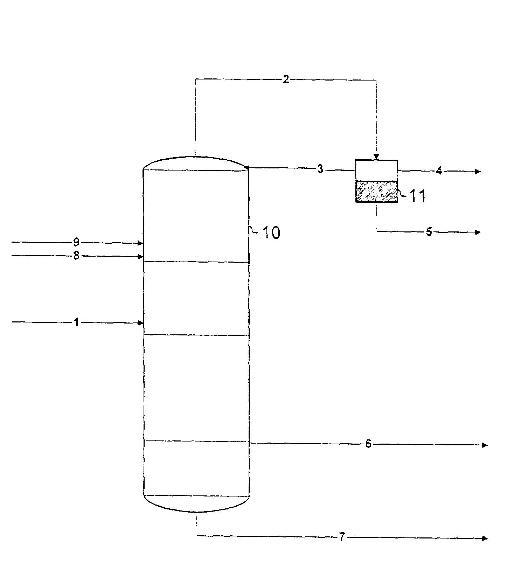

In Zeichnung 1 ist eine schematische Darstellung der Aufarbeitung von rohem, flüssigen Vinylacetat wiedergegeben. Das einzusetzende rohe, flüssige Vinylacetat (Strom 1) wird in eine Destillationskolonne 10 eingeleitet. Geeignete Destillationskolonnen sind beispielsweise solche, die 50 bis 110, vorzugsweise 55 bis 105 Böden enthalten und bei Normaldruck oder leicht erhöhtem Druck, beispielsweise bei 0,1 bis 0,5 MPa absolut, vorzugsweise jedoch bei Normaldruck, betrieben werden. Die Zugabe des rohen, flüssigen Vinylacetats kann dabei beispielsweise an einer Stelle erfolgen, die im mittleren Bereich der Kolonne 10, vorzugsweise zwischen der Mitte und dem untersten Drittel der Kolonne 10, liegt. Die Kolonne 10 wird so betrieben, daß man als Kopfprodukt (Strom 2) ein im wesentlichen Vinylacetat und Wasser enthaltendes gasförmiges Gemisch und als Sumpfprodukt (Strom 7) im wesentlichen Essigsäure erhält. Ferner entnimmt man einen Seitenstrom (Strom 6), in dem Ethylacetat angereichert ist. Vorzugsweise betreibt man die Kolonne 10 so, daß sich in der im Sumpf abgezogenen Essigsäure (Strom 7) ein Wassergehalt von 0,5 bis 6 Gew.-% und ein Vinylacetatgehalt von 1 bis 30 Gew.-ppm einstellt und der unterhalb des Zulaufs des Einsatzgemisches entnommene Seitenstrom (Strom 6) beispielsweise bis zu 15 Gew.-% Ethylacetat neben schwankenden Mengen an Vinylacetat, Essigsäure und Wasser enthält. Dieser Seitenstrom 6 kann flüssig oder gasförmig entnommen werden, vorzugsweise wird er in flüssiger Form entnommen. Das Kopfprodukt (Strom 2) wird kondensiert und in einem Phasentrenner 11 in eine obere mit Wasser gesättigte Vinylacetat-Phase und in eine mit Vinylacetat gesättigte untere Wasserphase separiert, die als Strom 5 abgeführt wird. Es kann ein Phasentrenner üblicher Bauart, z.B. ein Dekanter, verwendet werden. Nach erfolgter Phasentrennung wird ein Teil der mit Wasser gesättigten Vinylacetat-Phase als Rücklauf in die Destillationskolonne 10 zurückgeführt (Strom 3). Der Anteil des rückgeführten, mit Wasser gesättigten Vinylacetats kann dabei so reguliert werden, daß sich im Sumpf der Kolonne 10 der gewünschte Wassergehalt, beispielsweise im Bereich von 0,5 bis 6 Gew.-% einstellt. Hierfür ist im allgemeinem ein Rücklaufverhältnis (Verhältnis von zurückgeführtem zu entnommenem Vinylacetat) im Bereich von 1:1 bis 8:1 vorteilhaft. Das nicht zurückgeführte wassergesättigte Vinylacetat (Strom 4) wird abgetrennt und kann, falls erforderlich, noch weiter gereinigt werden. Das bei der Destillation anfallende Sumpfprodukt (Strom 7), das im allgemeinen zu über 90 Gew.-% Essigsäure enthält, kann in das Verfahren zur Herstellung von Vinylacetat zurückgeführt werden, gegebenenfalls nach Abtrennung von Verunreinigungen, z.B. Polymeren. Oberhalb der Zugabestelle des rohen, flüssigen Vinylacetats wird Wasser eingeleitet (Strom 8). Die Quelle, aus welcher das Wasser über Strom 8 eingeleitet wird, ist nicht kritisch. Das zugesetzte Wasser kann auch bis zu 5 Gew.-% organische Bestandteile, bezogen auf die Gesamtmenge von Strom 8, enthalten. Solche organischen Bestandteile sind beispielsweise Vinylacetat oder Ethylacetat. Die Mengen Wasser, die auf diese Weise eingeleitet werden, sind relativ gering, im allgemeinen 0,1 bis 5 Gew.-%, vorzugsweise 0,1 bis 2 Gew.-%, bezogen auf das eingesetzte rohe, flüssige Vinylacetat. Die bisher beschriebene Arbeitsweise, einschließlich der gegebenenfalls durchzuführenden weiteren Reinigung des abgetrennten Vinylacetats und der im Sumpf anfallenden Essigsäure ist beispielsweise aus der DE-AS 1 768 412 und EP-B-0-072 484 bekannt.

Die gemäß der vorliegenden Erfindung bei der Aufarbeitung von rohem, flüssigem Vinylacetat vorzunehmende Maßnahme besteht nun darin, daß man oberhalb der Zugabestelle des rohen, flüssigen Vinylacetats Wasser (Strom 8) in einer Menge einleitet, die nicht größer ist als die Menge, die zum Erreichen eines maximalen Wassergehaltes in den die Destillationskolonne 10 am Kopf verlassenden Gasen (Strom 2) erforderlich ist, und daß man oberhalb der Zugabestelle des rohen, flüssigem Vinylacetats Essigsäure (Strom 9) in einer Menge einleitet, die nicht größer ist als die Menge, die zum Erreichen eines maximalen Essigsäuregehaites in den die Destillationskolonne 10 am Kopf verlassenden Gasen (Strom 2) erforderlich ist.

Die Quelle, aus welcher Essigsäure über Strom 9 eingeleitet wird, ist nicht kritisch. Der Essigsäuregehalt im Strom 9 liegt im allgemeinen bei mehr als 80 Gew.-% und vorzugsweise bei mehr als 90 Gew.-%. Weitere Bestandteile von Strom 9 sind Wasser und gegebenenfalls geringe Mengen organischer Verbindungen, wie z.B. Ethylacetat.

Bereits geringe Mengen Wasser, im allgemeinen 0,1 bis 5 und vorzugsweise 0,1 bis 2 Gew.-%, bezogen auf eingesetztes rohes, flüssiges Vinylacetat, und Essigsäure, im allgemeinen 0,1 bis 60, vorzugsweise 0,1 bis 30 und besonders bevorzugt 0,1 bis 10 Gew.-%, bezogen auf eingesetztes rohes, flüssiges Vinylacetat, die auf diese Weise eingeleitet werden, lassen den Wassergehalt in den die Destillation am Kopf verlassenden Gasen (Strom 2) ansteigen und führen damit zu einer ausreichenden Entwässerung in der Destillation und bewirken eine Verringerung des Ethylacetatgehaltes in dem über den Kopf der Destillationskolonne 10 abgetrennten Vinylacetat. Diese Fahrweise bewirkt auch, daß der Vinylacetatgehalt in dem unterhalb der Zugabestelle des rohen, flüssigen Vinylacetats entnommenen Seitenstrom (Strom 6) reduziert wird. Dadurch wird mehr Vinylacetat über den Kopf der Destillationskolonne 10 gewonnen. Ein weiterer Vorteil des erfindungsgemäßen Verfahrens besteht darin, daß der Ethylacetatgehalt in dem Seitenstrom (Strom 6) erhöht wird und damit Vinylacetat über den Kopf der Kolonne 10 in einer höheren Reinheit gewonnen wird.

Die Wasserzugabe bewirkt, daß der Wassergehalt in dem die Destillation am Kopf verlassenden Gas (Strom 2) ansteigt, was zu einer ausreichenden Entwässerung in der Destillation führt. Wasser kann auf diese Weise bis zu einer solchen Menge eingesetzt werden, bis der Wassergehalt der die Destillationskolonne 10 am Kopf verlassenden Gase (Strom 2) maximal 7,3 Gew.-% beträgt. Es ist bekannt, daß das Vinylacetat/Wasser-Azeotrop einen maximalen Gehalt von 7,3 Gew.-% Wasser aufweisen kann (s. Advances in Chemistry, Series 116, Azeotropic Data III, American Chemical Society, Washington D.C., 1973). Dieser Wert kann aber bei der praktischen Durchführung des erfindungsgemäßen Verfahrens im allgemeinen nicht ganz erreicht werden. Die bei der praktischen Durchführung des erfindungsgemäßen Verfahrens maximal erreichbaren Wassergehalte in den die Destillationskolonne 10 am Kopf verlassenden Gasen (Strom 2) betragen etwa 6,8 Gew.-%. Vorzugsweise gibt man auf die erfindungsgemäße Weise gerade soviel Wasser zu, daß im Strom 2 der Wassergehalt 6,7 bis 6,9 Gew.-% beträgt.

Die Essigsäurezugabe bewirkt eine Abtrennung des Ethylacetats aus vinylacetat und Wasser. Dies ist zwar aus dem Stand der Technik (DE-OS-2 132 299) bekannt, doch müssen pro 100 g eingesetztem rohem, flüssigem Vinylacetat 60 g Essigsäure eingespeist werden.

Die erfindungsgemäß einzuleitenden Wasser- und Essigsäuremengen sind relativ gering. Sie betragen im allgemeinen 0,1 bis 5 Gew.-% Wasser, bezogen auf das eingesetzte rohen, flüssige Vinylacetat, und im allgemeinen 0,1 bis 60 Gew.-% Essigsäure, bezogen auf das eingesetzte rohe, flüssige Vinylacetat. Vorzugsweise beträgt die Wassermenge 0,1 bis 2 Gew.-%, bezogen auf das eingesetzte rohe, flüssige Vinylacetat. Die Essigsäuremenge beträgt vorzugsweise 0,1 bis 30 Gew.-%, und besonders bevorzugt 0,1 bis 10 Gew.-%, bezogen auf das eingesetzte rohe, flüssige Vinylacetat. Welche Wassermengen und Essigsäuremengen im konkreten Einzelfall notwendig sind, um in den die Destillation am Kopf verlassenden Gasen (Strom 2) den möglichst hohen Wassergehalt und gleichzeitig den möglichst niedrigen Ethylacetatgehalt zu erzielen sowie gleichzeitig in dem die Destillation verlassenden Seitenstrom (Strom 6) den möglichst hohen Ethylacetatgehalt zu erreichen, kann gegebenenfalls durch einfache Versuche ermittelt werden. Diese Mengen hängen z.B. von den Mengen an Wasser, Essigsäure und Vinylacetat in dem flüssigen, eingesetzten, rohen Vinylacetat ab.

Die erfindungsgemäß zuzusetzenden Mengen an Essigsäure und Wasser können in flüssiger Form oder als Dampf in die Destillationskolonne 10 eingeleitet werden. Die Essigsäure- und Wasserzugabe erfolgen oberhalb der Zugabestelle des rohen, flüssigen Vinylacetats. Werden Essigsäure und Wasser unterhalb des Zulaufs des rohen, flüssigen Vinylacetats oder zusammen mit diesem eingeleitet, so beobachtet man in der Destillation keine zufriedenstellende. Entwässerung und keine zufriedenstellende Verringung des Ethylacetat-Gehaltes in dem aus dem Kopfprodukt der Destillation abgetrennten Vinylacetat. Für das erfindungsgemäße Verfahren ist es daher wesentlich, die erfindungsgemäße Einleitung von Essigsäure und Wasser oberhalb der Zugabe des rohen, flüssigen Vinylacetats vorzunehmen, im allgemeinen 2 bis 50 Böden, vorzugsweise 10 bis 35 Böden oberhalb der Zugabestelle des rohen, flüssigen Vinylacetats. Essigsäure und Wasser können auf dem gleichen Boden eingeleitet werden, beide Stoffe können aber auch auf verschiedenen Böden eingeleitet werden. Welche Zugabestellen für die geringen Mengen Essigsäure und Wasser oberhalb der Zugabestelle des rohen, flüssigen Vinylacetats im konkreten Einzelfall notwendig sind, um in den die Destillation am Kopf verlassenden Gasen (Strom 2) einen möglichst hohen Wassergehalt und gleichzeitig einen möglichst niedrigen Ethylacetatgehalt sowie gleichzeitig in dem die Destillation verlassenden Seitenstrom (Strom 6) einen möglichst hohen Ethylacetatgehalt zu erreichen, kann gegebenenfalls durch einfache Versuche ermittelt werden.

Die erfindungsgemäße Arbeitsweise hat folgende Vorteile:
- Die Trennung zwischen Vinylacetat und Ethylacetat wird verbessert. Das bedeutet, man kann bei unverändertem Vinylacetat-Rücklauf ein aus dem Kopfprodukt der Destillation abgetrenntes Vinylacetat (Strom 4) erhalten, das bis zu etwa 40% weniger Ethylacetat als bisher enthält. Soll der Ethylacetat-Gehalt im aus dem Kopfprodukt der Destillation abgetrennten Vinylacetat in der Größenordnung wie bisher liegen, so gestattet das erfindungsgemäße Verfahren die Durchführung der Destillation bei einem geringeren Vinylacetat-Rücklauf und/oder den Einsatz eines flüssigen, rohen Vinylacetats mit einem höheren Ethylacetat-Gehalt und/oder die Verwendung einer kürzeren Destillationskolonne.
- Die Menge von Vinylacetat im Ethylacetat-Seitenabzug (Strom 6) wird reduziert. Das bedeutet, daß man bis zu 60% weniger Vinylacetat über den Seitenabzug ausschleust bei gleicher Menge von Ethylacetat in diesem Seitenabzug. Dadurch wird der Verlust an Vinylacetat erheblich reduziert.

Besonders vorteilhaft ist es, daß die erfindungsgemäß erzielbaren Verbesserungen ohne Erhöhung des Aufwandes für die Destillation, z.B. in Bezug auf Bodenzahl, Rücklaufmengen und Energiebedarf, erreicht werden. Es ist überraschend, daß diese beiden Effekte, nämlich die Verringerung des Ethylacetat-Gehaltes in dem aus dem Kopfprodukt der Destillation anfallenden Vinylacetats (Strom 4) und die Verringerung des Vinylacetat-Gehaltes in dem über den Seitenabzug ausgeschleusten Ethylacetat (Strom 6), durch eine einfache Maßnahme, nämlich der erfindungsgemäßen Wasser-und Essigsäurezugabe, erzielt werden können.

Die folgenden Beispiele sollen das erfindungsgemäße Verfahren erläutern.

### Beispiele

Eine schematische Beschreibung der im folgenden gegebenen Beispiele sind in Zeichnung 1 zu sehen.

### Beispiel 1

### (Vergleichsbeispiel)

In die Destillationskolonne 10 mit 98 Böden wurden pro Stunde 62 t eines Einsatzgemisches folgender Zusammensetzung auf den 48. Boden über dem Kolonnenfuß gegeben (Strom 1).

| | |
|---|---|
| Vinylacetat | 25,00 Gew.-% |
| Ethylacetat | 0,07 Gew.-% |
| Wasser | 7,00 Gew.-% |
| Essigsäure | 67,50 Gew.-% |
| Rest | 0,43 Gew.-% |

Das Einsatzgemisch enthielt als Rest Methylacetat, Acetaldehyd und anderen Verunreinigungen, z.B. Diacetate oder Polymere, jeweils in Mengen von unter 1000 Gew.-ppm. Das Kopfprodukt (Strom 2) wurde kondensiert, auf 26°C abgekühlt und in einen Phasenabscheider 11 geleitet. Von der Vinylacetatphase wurden 33,5 t/h als Rücklauf auf den Kopf der Destillationskolonne so gepumpt (Strom 3), der Rest wurde entnommen (Strom 4). Die Zusammensetzung des abgetrennten Vinylacetats (Strom 4) war wie folgt:

| | |
|---|---|
| Ethylacetat | 270 Gew.-ppm |
| Wasser | 1,20 Gew.-% |
| Vinylacetat | ergänzend auf 100 Gew.-% |

Eine wässerige Phase (Strom 8) mit einer Menge von 0,7 t/h wurde auf den 69. Boden über dem Kolonnenfuß gepumpt. Die Zusammensetzung der wässerigen Phase (Strom 8) war wie folgt:

| | |
|---|---|
| Wasser | 98,0 Gew.-% |
| Vinylacetat | 2,0 Gew.-% |

Die aus dem Phasenabscheider 11 entnommene Wassermenge (Strom 5) betrug 2,2 t/h. Die Zusammensetzung dieser Wasserphase war wie folgt:

| | |
|---|---|
| Wasser | 98,0 Gew.-% |
| Vinylacetat | 2,0 Gew.-% |

Vom 17. Boden über dem Kolonnenfuß der Kolonne 10 wurde ein flüssiger Seitenstrom (Strom 6) in einer Menge von 3,6 t/h mit der Zusammensetzung

| | |
|---|---|
| Vinylacetat | 2,0 Gew.-% |
| Ethylacetat | 1,0 Gew.-% |
| Wasser | 10,0 Gew.-% |
| Essigsäure | 87,0 Gew.-% |

entnommen.

Ein flüssiger Strom, der im wesentlichen aus Essigsäure besteht (Strom 9), wurde in einer Menge von 3,5 t/h auf den 11. Boden über dem Kolonnenfuß gepumpt. Die Zusammensetzung dieses Essigsäurestroms war wie folgt:

| | |
|---|---|
| Wasser | 10,1 Gew.-% |
| Essigsäure | 89,9 Gew.-% |

### Beispiel 2

Die in Beispiel 1 beschriebene Destillationskolonne 10 wurde unter den gleichen Bedingungen wie in Beispiel 1 betrieben. Im Gegensatz zu Beispiel 1 wurde in diesem Beispiel der Essigsäurestrom (Strom 9) mit einer Menge von 3,5 t/h auf den 69. Boden über dem Kolonnenfuß geführt. Die Zusammensetzung des abgetrennten Vinylacetats (Strom 4) war wie folgt:

| | |
|---|---|
| Ethylacetat | 200 Gew.-ppm |
| Wasser | 1,20 Gew.-% |
| Vinylacetat | ergänzend auf 100 Gew.-% |

Vom 17. Boden über dem Kolonnenfuß der Kolonne 10 wurde ein flüssiger Seitenstrom (Strom 6) in einer Menge von 3,6 t/h mit der Zusammensetzung

| | |
|---|---|
| Vinylacetat | 1,00 Gew.-% |
| Ethylacetat | 1,00 Gew.-% |
| Wasser | 10,00 Gew.-% |
| Essigsäure | 88,00 Gew.-% |

entnommen.

Wie aus Beispiel 2 ersichtlich, führt die erfindungsgemäße Wasser- und Essigsäurezugabe zu einer Verringerung des Ethylacetatgehalts in dem aus dem Kopfprodukt der Destillation anfallenden Vinylacetat (Strom 4) und zu einer Verringerung des Vinylacetat-Gehalts in dem über den Seitenabzug (Strom 6) ausgeschleusten Ethylacetat.

## Patentansprüche

1. Verfahren zur Aufarbeitung von rohem, flüssigem Vinylacetat, das Essigsäure und Wasser, sowie Ethylacetat und gegebenenfalls weitere kleinere Mengen anderer Verunreinigungen enthält, durch Destillation, wobei man als Kopfprodukt ein im wesentlichen Vinylacetat und Wasser enthaltendes Gemisch und als Sumpfprodukt im wesentlichen Essigsäure erhält und einen Seitenstrom entnimmt, in dem Ethylacetat angereichert ist, und wobei man das Kopfprodukt kondensiert und nach Phasentrennung einen Teil der Vinylacetat-Phase als Rücklauf in die Destillationskolonne zurückführt, **dadurch gekennzeichnet, daß** man in die Destillationskolonne oberhalb der Zugabestelle des rohen, flüssigen Vinylacetats 0,1 bis 5 Gew.-% Wasser, bezogen auf das eingesetzte rohe, flüssige Vinylacetat, und 0,1 bis 60 Gew.-% Essigsäure, bezogen auf das eingesetzte rohe, flüssige Vinylacetat, einleitet.

2. Verfahren nach Anspruch 1, **dadurch gekennzeichnet, daß** man rohes, flüssiges Vinylacetat aufarbeitet, das 12 bis 30 Gew.-% Vinylacetat, 3 bis 10 Gew.-% Wasser, 0,01 bis 0,2 Gew.-% Ethylacetat und ergänzend auf 100 Gew.-% Essigsäure enthält.

3. Verfahren nach Ansprüchen 1 und 2, **dadurch gekennzeichnet, daß** das rohe, flüssige Vinylacetat zusätzlich Diacetate, Polymere, Acetaldehyd, und/oder Methylacetat, jeweils in Mengen unter 0,5 Gew.-% enthält.

4. Verfahren nach Ansprüchen 1 bis 3, **dadurch gekennzeichnet, daß** man gerade soviel Wasser einleitet, daß der Wassergehalt in den die Destillationskolonne am Kopf verlassenden Gasen im Bereich von 6,7 bis 7,3 Gew.-% liegt.

5. Verfahren nach Ansprüchen 1 bis 4, **dadurch gekennzeichnet, daß** man Wasser in flüssiger Form einleitet.

6. Verfahren nach Ansprüchen 1 bis 4, **dadurch gekennzeichnet, daß** man Wasser in Form von Wasserdampf einleitet.

7. Verfahren nach Ansprüchen 1 bis 6, **dadurch gekennzeichnet, daß** man Wasser 2 bis 50 Böden oberhalb der Zugabestelle des rohen, flüssigen Vinylacetats einleitet.

8. Verfahren nach Ansprüchen 1 bis 7, **dadurch gekennzeichnet, daß** man Wasser 10 bis 35 Böden oberhalb der Zugabestelle des rohen, flüssigen Vinylacetats einleitet.

9. Verfahren nach Ansprüchen 1 bis 8, **dadurch gekennzeichnet, daß** man in die Destillationskolonne oberhalb der Zugabestelle des rohen, flüssigen Vinylacetats 0,1 bis 30 Gew.-% Essigsäure, bezogen auf das eingesetzte rohe, flüssige Vinylacetat, einleitet.

10. Verfahren nach Ansprüchen 1 bis 9, **dadurch gekennzeichnet, daß** man in die Destillationskolonne oberhalb der Zugabestelle des rohen, flüssigen Vinylacetats 0,1 bis 10 Gew.-% Essigsäure, bezogen auf das eingesetzte rohe, flüssige Vinylacetat, einleitet.

11. Verfahren nach Ansprüchen 1 bis 10, **dadurch gekennzeichnet, daß** man Essigsäure in flüssiger Form einleitet.

12. Verfahren nach Ansprüchen 1 bis 10, **dadurch gekennzeichnet, daß** man Essigsäure in Form von Dampf einleitet.

13. Verfahren nach Ansprüchen 1 bis 12, **dadurch gekennzeichnet, daß** man Essigsäure 2 bis 50 Böden oberhalb der Zugabestelle des rohen, flüssigen Vinylacetats einleitet.

14. Verfahren nach Ansprüchen 1 bis 13, **dadurch gekennzeichnet, daß** man Essigsäure 10 bis 35 Böden oberhalb der Zugabestelle des rohen, flüssigen Vinylacetats einleitet.

15. Verfahren nach Ansprüchen 1 bis 14, **dadurch gekennzeichnet, daß** man Essigsäure und Wasser auf dem gleichen der 2 bis 50 Böden oberhalb der Zugabestelle des rohen, flüssigen Vinylacetats einleitet.

16. Verfahren nach Ansprüchen 1 bis 15, **dadurch gekennzeichnet, daß** man Essigsäure und Wasser auf dem gleichen der 10 bis 35 Böden oberhalb der Zugabestelle des rohen, flüssigen Vinylacetats einleitet.

## Claims

1. A process for working up crude, liquid vinyl acetate containing acetic acid and water and also ethyl acetate and possibly small amounts of other impurities by distillation, giving a mixture essentially consisting of vinyl acetate and water as overhead product and essentially acetic acid as bottom product and a side stream being taken off in which ethyl acetate is enriched, and the overhead product being condensed and, after phase separation, some of the vinyl acetate phase being recirculated as reflux to the distillation column, which comprises introducing 0.1 to 5% by weight of water, based on the crude, liquid vinyl acetate feed, and 0.1 to 60% by weight of acetic acid, based on the crude, liquid vinyl acetate feed, into the distillation column above the feed point of the crude, liquid vinyl acetate.

2. The process as claimed in claim 1, wherein crude, liquid vinyl acetate which contains 12 to 30% by weight of vinyl acetate, 3 to 10% by weight of water, 0.01 to 0.2% by weight of ethyl acetate and acetic acid to make up 100% by weight is worked up.

3. The process as claimed in claims 1 and 2, wherein the crude, liquid vinyl acetate additionally contains diacetates, polymers, acetaldehyde and/or methyl acetate, each in amounts below 0.5% by weight.

4. The process as claimed in claims 1 to 3, wherein just enough water is introduced so that the water content in the gases leaving the distillation column at the top is in the range from 6.7 to 7.3% by weight.

5. The process as claimed in claims 1 to 4, wherein water is introduced in liquid form.

6. The process as claimed in claims 1 to 4, wherein water is introduced in the form of steam.

7. The process as claimed in claims 1 to 6, wherein water is introduced 2 to 50 plates above the feed point of the crude, liquid vinyl acetate.

8. The process as claimed in claims 1 to 7, wherein water is introduced 10 to 35 plates above the feed point of the crude, liquid vinyl acetate.

9. The process as claimed in claims 1 to 8, wherein 0.1 to 30% by weight of acetic acid, based on the crude, liquid vinyl acetate used, is introduced into the distillation column above the feed point of the crude, liquid vinyl acetate.

10. The process as claimed in claims 1 to 9, wherein 0.1 to 10% by weight of acetic acid, based on the crude, liquid vinyl acetate used, is introduced into the distillation column above the feed point of the crude, liquid vinyl acetate.

11. The process as claimed in claims 1 to 10, wherein acetic acid is introduced in liquid form.

12. The process as claimed in claims 1 to 10, wherein acetic acid is introduced in the form of vapor.

13. The process as claimed in claims 1 to 12, wherein acetic acid is introduced 2 to 50 plates above the feed point of the crude, liquid vinyl acetate.

14. The process as claimed in claims 1 to 13, wherein acetic acid is introduced 10 to 35 plates above the feed point of the crude, liquid vinyl acetate.

15. The process as claimed in claims 1 to 14, wherein acetic acid and water are introduced on the same plate of the 2 to 50 plates above the feed point of the crude, liquid vinyl acetate.

16. The process as claimed in claims 1 to 15, wherein acetic acid and water are introduced on the same plate of the 10 to 35 plates above the feed point of the crude, liquid vinyl acetate.

## Revendications

1. Procédé de traitement d'acétate de vinyle liquide brut qui contient de l'acide acétique et de l'eau, ainsi que de l'acétate d'éthyle et éventuellement de petites quantités supplémentaires d'autres impuretés, par distillation, où on obtient comme produit de tête un mélange contenant essentiellement de l'acétate de vinyle et de l'eau et comme produit de fond essentiellement de l'acide acétique et on retire un courant latéral dans lequel l'acétate d'éthyle se trouve enrichi, et où on condense le produit de tête et, après séparation des phases, on renvoie dans la colonne de distillation une partie de la phase d'acétate de vinyle sous forme de reflux, **caractérisé en ce que** l'on introduit dans la colonne de distillation, au-dessus de l'endroit d'introduction de l'acétate de vinyle liquide brut, 0,1 à 5 % en masse d'eau, par rapport à l'acétate de vinyle liquide brut utilisé, et 0,1 à 60 % en masse d'acide acétique, par rapport à l'acétate de vinyle liquide brut utilisé.

2. Procédé selon la revendication 1, **caractérisé en ce que** l'on traite un acétate de vinyle liquide brut qui contient 12 à 30 % en masse d'acétate de vinyle, 3 à 10 % en masse d'eau, 0,01 à 0,2 % en masse d'acétate d'éthyle et le complément à 100 % en masse d'acide acétique.

3. Procédé selon les revendications 1 et 2, **caractérisé en ce que** l'acétate de vinyle liquide brut contient en outre des diacétates, des polymères, de l'acétaldéhyde et/ou de l'acétate de méthyle, dans chaque cas en des quantités inférieures à 0,5 % en masse.

4. Procédé selon les revendications 1 à 3, **caractérisé en ce que** l'on introduit de l'eau en une quantité telle que la teneur en eau des gaz quittant la colonne de distillation en tête est située dans le domaine de 6,7 à 7,3 % en masse.

5. Procédé selon les revendications 1 à 4, **caractérisé en ce que** l'on introduit l'eau sous forme liquide.

6. Procédé selon les revendications 1 à 4, **caractérisé en ce que** l'on introduit l'eau sous forme de vapeur d'eau.

7. Procédé selon les revendications 1 à 6, **caractérisé en ce que** l'on introduit l'eau 2 à 50 plateaux au-dessus de l'endroit d'introduction de l'acétate de vinyle liquide brut.

8. Procédé selon les revendications 1 à 7, **caractérisé en ce que** l'on introduit l'eau 10 à 35 plateaux au-dessus de l'endroit d'introduction de l'acétate de vinyle liquide brut.

9. Procédé selon les revendications 1 à 8, **caractérisé en ce que** l'on introduit dans la colonne de distillation, au-dessus de l'endroit d'introduction de l'acétate de vinyle liquide brut, 0,1 à 30 % en masse d'acide acétique, par rapport à l'acétate de vinyle liquide brut utilisé.

10. Procédé selon les revendications 1 à 9, **caractérisé en ce que** l'on introduit dans la colonne de distillation, au-dessus de l'endroit d'introduction de l'acétate de vinyle liquide brut, 0,1 à 10 % en masse d'acide acétique, par rapport à l'acétate de vinyle liquide brut introduit.

11. Procédé selon les revendications 1 à 10, **caractérisé en ce que** l'on introduit l'acide acétique sous forme liquide.

12. Procédé selon les revendications 1 à 10, **caractérisé en ce que** l'on introduit l'acide acétique sous forme de vapeur.

13. Procédé selon les revendications 1 à 12, **caractérisé en ce que** l'on introduit l'acide acétique 2 à 50 plateaux au-dessus de l'endroit d'introduction de l'acétate de vinyle liquide brut.

14. Procédé selon les revendications 1 à 13, **caractérisé en ce que** l'on introduit l'acide acétique 10 à 35 plateaux au-dessus de l'endroit d'introduction de l'acétate de vinyle liquide brut.

15. Procédé selon les revendications 1 à 14, **caractérisé en ce que** l'on introduit l'acide acétique et l'eau sur le même plateau parmi les 2 à 50 plateaux au-dessus de l'endroit d'introduction de l'acétate de vinyle liquide brut.

16. Procédé selon les revendications 1 à 15, **caractérisé en ce que** l'on introduit l'acide acétique et l'eau sur le même plateau parmi les 10 à 35 plateaux au-dessus de l'endroit d'introduction de l'acétate de vinyle liquide brut.
